# EUROPEAN PATENT SPECIFICATION

(11) **EP 3 974 015 B1**
(45) Date of publication and mention of the grant of the patent: **25.03.2026**
(21) Application number: 21207880.2
(22) Date of filing: 12.05.2009
(51) Int. Cl.: A61M 16/06, A62B 9/00, A61M 16/08

(54) **INTERFACE**
SCHNITTSTELLE
INTERFACE

(30) Priority: 12.05.2008 US 52362 P
(43) Date of publication of application: 30.03.2022
(62) Divisional of application: 19197671.1
(73) Proprietor: Fisher & Paykel Healthcare Limited, East Tamaki Auckland 2013 (NZ)
(72) Inventor: Gradon, Lewis George, 2013 Auckland (NZ); Salmon, Andrew Paul Maxwell, 2013 Auckland (NZ); Siew, Silas Sao Jin, 2013 Auckland (NZ)
(74) Representative: Hoffmann Eitle

(56) References cited:
- EP-A2- 0 747 078
- EP-A2- 2 022 528
- WO-A1-98/18514
- AU-A4- 2005 100 738
- US-A1- 2003 196 656
- US-A1- 2006 060 200
- US-A1- 2007 125 385

## Description

### FIELD OF THE INVENTION

The present invention relates to interfaces for providing a supply of pressurised gas to a recipient via the nasal passages. The invention may be included in appliances that also supply gas to the oral passages, for example by incorporation into a broader full face mask.

### SUMMARY OF THE PRIOR ART

The prior art includes a wide variety of interfaces for supplying gases to a recipient. The following are examples.

The prior art includes a nasal mask that can be used for supplying gases to a recipient. The nasal mask includes a perimeter seal that seals across, down each cheek alongside the nose and along the surface of the upper lip. The entire enclosed space is pressurised and the recipient may inhale the pressurised gas from the enclosed space. An example is the Flexifit 405 nasal mask sold by Fisher & Paykel Healthcare.

The prior art includes a full face mask. The full face mask includes a perimeter seal that extends across the bridge of the nose downward along each cheek beside the nose to the jaw and along the jaw below the lower lip. The perimeter thereby encloses both the nose and mouth. The entire space within the mask frame is pressurised. The recipient may breathe the pressurised gas from the space through either the nose or mouth. An example is the Flexifit 431 interface sold by Fisher & Paykel Healthcare.

The prior art includes an oral interface including an oral appliance that fits within the user's mouth. An example is the Fisher & Paykel Healthcare Oracle interface.

The prior art includes a nasal pillows interface in which headgear retains a soft plenum in the vicinity of the user's nose. A pair of flexible protrusions engage against the nares of the recipient. Typically, the protrusions are able to axially compress and have a lateral freedom of movement relative to the supporting cushion. An example is the ResMed Swift nasal pillows interface.

The prior art includes a nasal cannula interface. The nasal canal interface includes a plenum portion that rests against the upper lip of the user and a pair of prongs. Each prong extends into the nostril of the user. An example is the Nasal-Aire interface made by Innomed.

Interfaces such as these are frequently used for delivering pressurised gases to a person being treated for obstructive sleep apnea (OSA) or other sleep disorders. These users typically wear the interface in a home sleeping environment. Comfort and effective sealing even under conditions of patient movement are major considerations.

US 2003/196656 A1 relates to a respiratory mask assembly for delivering breathable gas to a patient including a frame having having a front surface and a rear surface adapted in use to face the patient.

US 2006/060200 A1 relates to a patient interface including a support body having a cushion and cheek interfaces.

EP 0 747 078 A2 relates to a miniature nasal mask for supplying breathing gas to a human user through an interface with the user's nares, the interface being maintained by a contact seal which engages the user's face only within an area between the tip and immediately adjacent lateral flanks of the user's nose adjacent to the nares, and the user's upper lip.

US 2007/125385 A1 relates to a patient interface that reliably and comfortably seals a user's face by providing an oral-nasal mask that includes an integrated nasal interface.

WO 98/18514 A1 relates to a nasal mask useful for patients suffering from respiratory ailments such as sleep apnea syndrome.

EP 2 022 528 A2 relates to a patient interface for delivering breathable gas to a patient including a nasal prong assembly including a pair of nasal prongs structured to sealingly communicate with nasal passages of a patient's nose in use and headgear to maintain the nasal prong assembly in a desired position on the patient's face.

AU 2005 100 738 A4 relates to a patient interface device that includes a frame sized and configured to span at least a portion of a patient's face while remaining below the patient's eyes when the patient interface device is donned by the patient.

The term "comprising" as used in this specification means "consisting at least in part of". When interpreting each statement in this specification that includes the term "comprising", features other than that or those prefaced by the term may also be present. Related terms such as "comprise" and "comprises" are to be interpreted in the same manner.

### SUMMARY OF THE INVENTION

An object of the present invention is to provide an interface which will at least provide the industry and users with useful choice.

The present invention is according to the interface as according to independent claim 1. Other optional preferred features are recited in the dependent claims.

In a first aspect the technology consists in a patient interface comprising a supple envelope, bag or balloon, with an air supply aperture and a pair of protruding nostril locators protruding from the envelope, each nasal locator including an outlet aperture, the envelope, bag or balloon inflating under internal pressure from a pressurised gases supply and when pressed against the face of a user, creating a seal with the nose or face of the user in addition to any seal provided by the nasal locators.

According to a further aspect the envelope has at least two integral connectors to attach a head strap to the envelope.

According to a further aspect the interface includes a frame having a gases inlet for connection with a gases supply conduit and a gases outlet including a protruding lip, where the gases supply aperture of the envelope is engaged or engagable over the protruding lip of the frame.

According to a further aspect the frame has at least two integral connectors to attach a head strap to the frame.

According to a further aspect the frame includes a strap to stabilise the envelope, the strap extending above the frame.

According to a further aspect the strap is integrally formed with the frame.

According to a further aspect the strap is separate to the frame yet fastened to said frame.

According to a further aspect the envelope includes an elongate strip integrally formed in the envelope to stabilise the envelope, the strip extending in an arc above the frame.

According to a further aspect the envelope, when inflated without stretch, will not pass through a circular aperture of 40mm diameter without contact.

According to a further aspect the envelope collapses when not inflated.

According to a further aspect the envelope or the body of the envelope, in use, forms a substantially continuous seal against the user's nose and face that surrounds the nostril locators.

According to a further aspect the envelope wraps completely about the user's nose.

According to a further aspect the envelope has side portions that extend completely about the sides of the user's nose and at least partially over the user's cheeks.

According to a further aspect the envelope has a cut out in the nasal bridge region of the user.

According to a further aspect wherein, in use, the side wall of the envelope rolls to accommodate movement of the mask frame relative to the nose, wherein rolls means that the portion of the envelope adjacent to the frame reduces at one side and extends at the other, and the corresponding portions adjacent the face extend at one side and reduce at the other.

According to a further aspect the envelope allows for this movement in any direction relative to the nose.

According to a further aspect the envelope allows for a roll of at least 10mm without exposing the nostril locators.

According to a further aspect substantially the entire envelope, except for a region immediately adjacent and including the nostril locators and immediately adjacent and including the inlet opening, is supple, and the region immediately adjacent and including the area immediately adjacent the inlet opening has any suitable stiffness.

According to a further aspect the region immediately adjacent the inlet opening is stiffer than the rest of the envelope.

According to a further aspect the region immediately adjacent the nostril locators is stiffer than the rest of the envelope.

According to a further aspect a connector is engaged with or formed in the interface frame.

According to a further aspect the interface includes a short length of supple breathing conduit that is connectable to the frame.

According to a further aspect the interface includes a including a short length of breathable conduit that is connectable to the frame.

According to a further aspect the interface includes a lanyard or tether connected to a length of breathing conduit connected to the frame.

According to a further aspect the lanyard or tether is connected to the conduit at a location between 10cm and 50cm from the frame.

According to a further aspect the interface includes a frame supporting the envelope, the frame including at least one soft or flexible portion at the perimeter.

According to a further aspect the frame includes at least a pair of extended support members at the perimeter, the support members located either side of the frame such that they may, but do not necessarily, contact portions of the face of the user in use.

According to a further aspect the interface includes at least one strap extending from one side portion of the frame to the other side portion of the frame, the strap being sufficient to pass around the back of the head of the user.

According to a further aspect the strap is adjustable in length.

According to a further aspect each end of the strap is bifurcated and the two limbs of the bifurcated end connect to the side portion of the frame at spaced apart locations.

According to a further aspect the interface includes a bias flow outlet in one or more of the envelope, a frame or connector.

According to a further aspect the envelope is inflated and holds its shape against the force of gravity with an elevated internal pressure equivalent to 3cm H₂O.

In a further aspect the technology consists in an interface that includes an inflatable envelope (that could also be referred to as a bag or balloon) having a supple wall structure. The inflatable envelope has a pair of locating protrusions that engage in the nostrils of the user. The locating protrusions supply gases flow to the user from inside the envelope. The envelope is so supple, and of sufficient dimension and shape, that when the inflated envelope is pressed against the face of a user, with the locating protrusions engaged in the nostrils of the user, the envelope contacts the surfaces of the user's face (the nose, the upper lip and the cheeks) and provides a seal.

To those skilled in the art to which the invention relates, many changes in construction and widely differing embodiments and applications of the invention will suggest themselves without departing from the scope of the invention as defined in the appended claims. The disclosures and the descriptions herein are purely illustrative and are not intended to be in any sense limiting.

In this specification where reference has been made to patent specifications, other external documents, or other sources of information, this is generally for the purpose of providing a context for discussing the features of the invention. Unless specifically stated otherwise, reference to such external documents is not to be construed as an admission that such documents, or such sources of information, in any jurisdiction, are prior art, or form part of the common general knowledge in the art.

### BRIEF DESCRIPTION OF THE DRAWINGS

Preferred forms of the interface will be described with reference to the accompanying drawings. One exemplary form will be described with reference to Figures 1 and 2. Behavioural and sealing characteristics of this example will be described with reference to Figures 3 to 5. Additional examples of the form of the inflating envelope will be described with reference to Figures 6 to 8. An additional example of an interface with an alternative frame is described with reference to Figure 9. A further example of an interface with an alternative envelope and frame is described with reference to Figures 10 to 12.

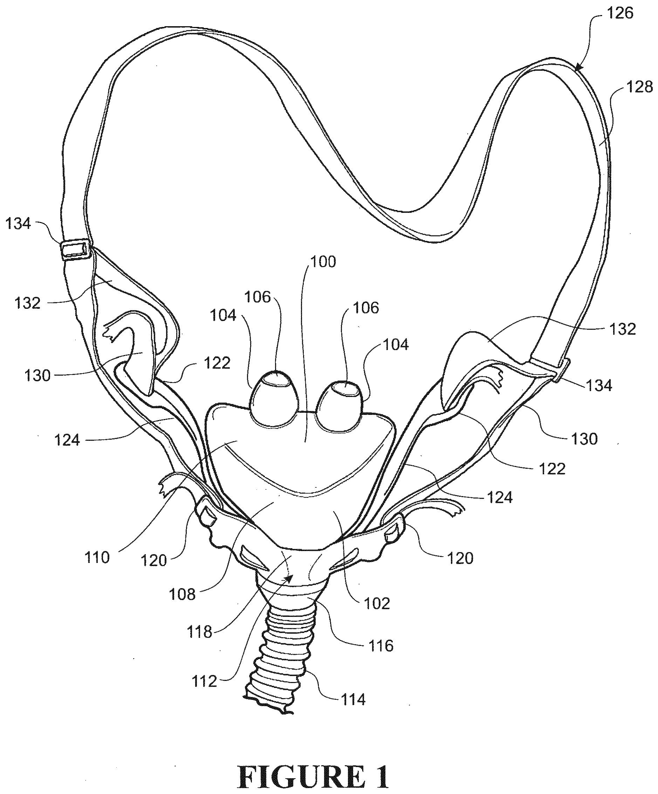

**Figure 1** is a view from above of an example interface including inflatable envelope, supporting frame, and straps.

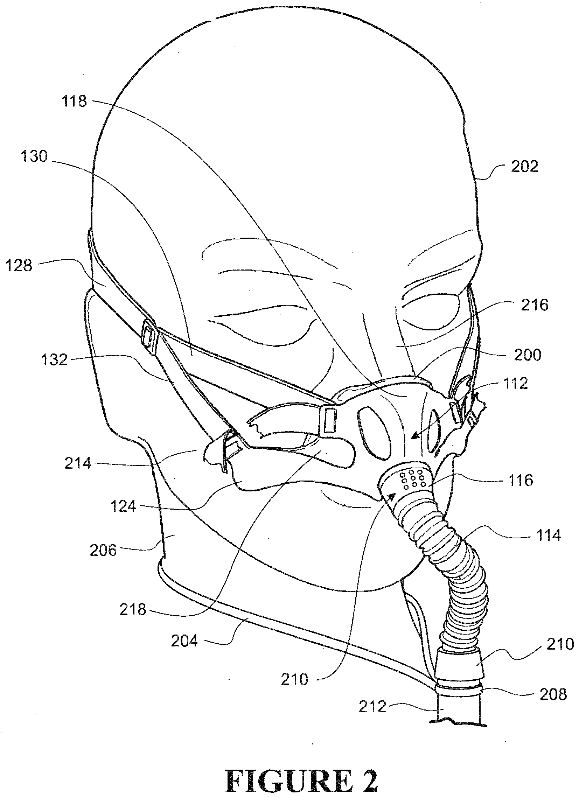

**Figure 2** is an illustration showing the interface of Figure 1 in position on the head of a user.

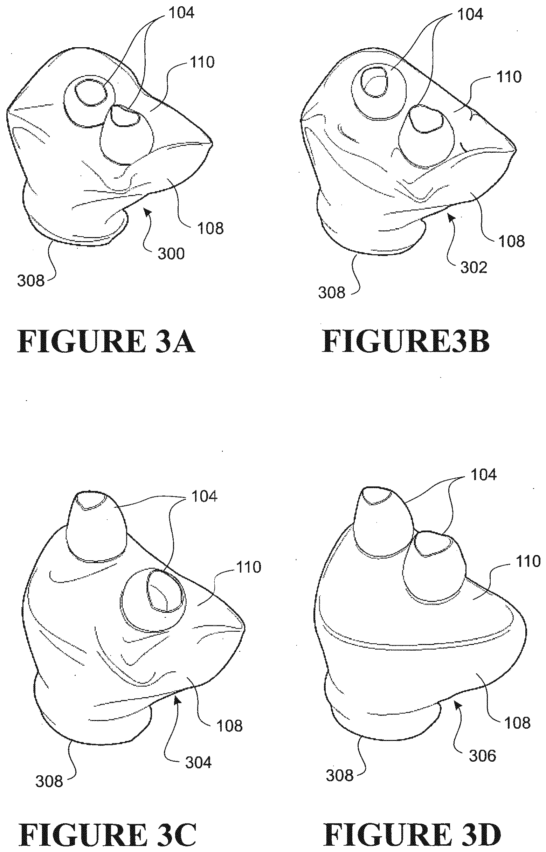

**Figures 3A** to **3D** are a sequence of illustrations showing inflation of the inflatable envelope. Figure 3A shows the seal limp and deflated. Figures 3B and 3C show the envelope with progressively greater inflation. Figure 3D shows the envelope inflated.

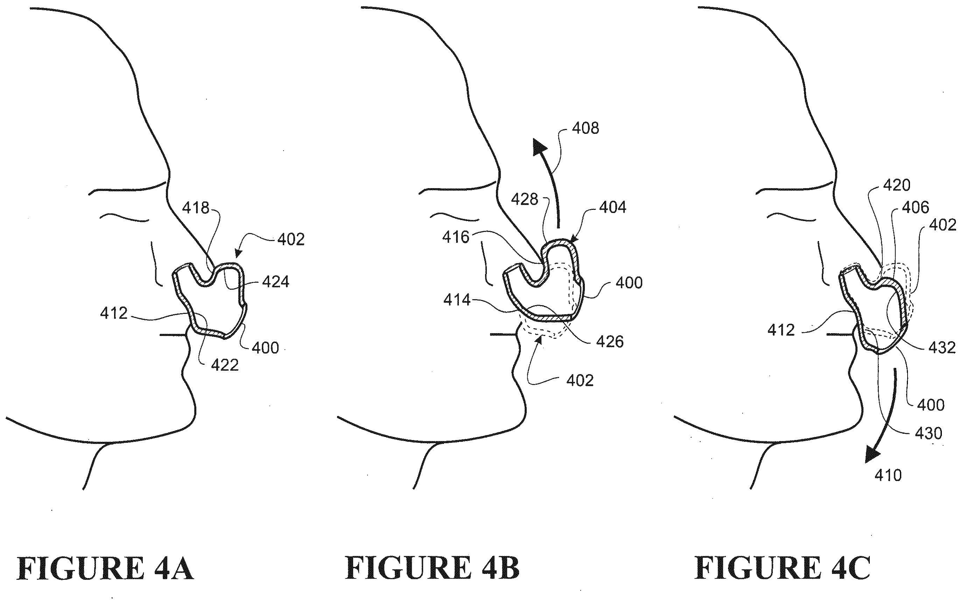

**Figures 4A** to **4C** show how the envelope retains a seal despite substantial vertical movement. Figure 4A shows an intended position on the user's face. Figure 4B shows a less than ideal position that might occur if the frame was moved upward. Figure 4C shows a less than ideal position that might occur if the frame was moved downward.

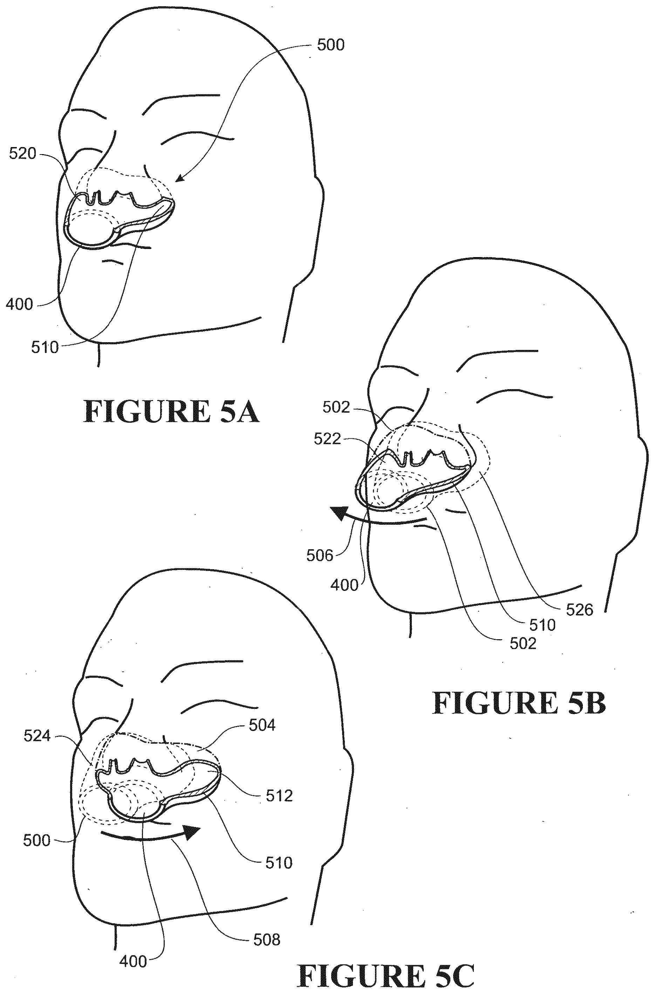

**Figures 5A to 5C** show how the envelope retains a seal despite substantial lateral movement. Figure 5A shows an intended position on the user's face. Figure 5B shows a less than ideal position that might occur if the frame was moved to the user's right. Figure 5C shows a less than ideal position that might occur if the frame was moved to the user's left.

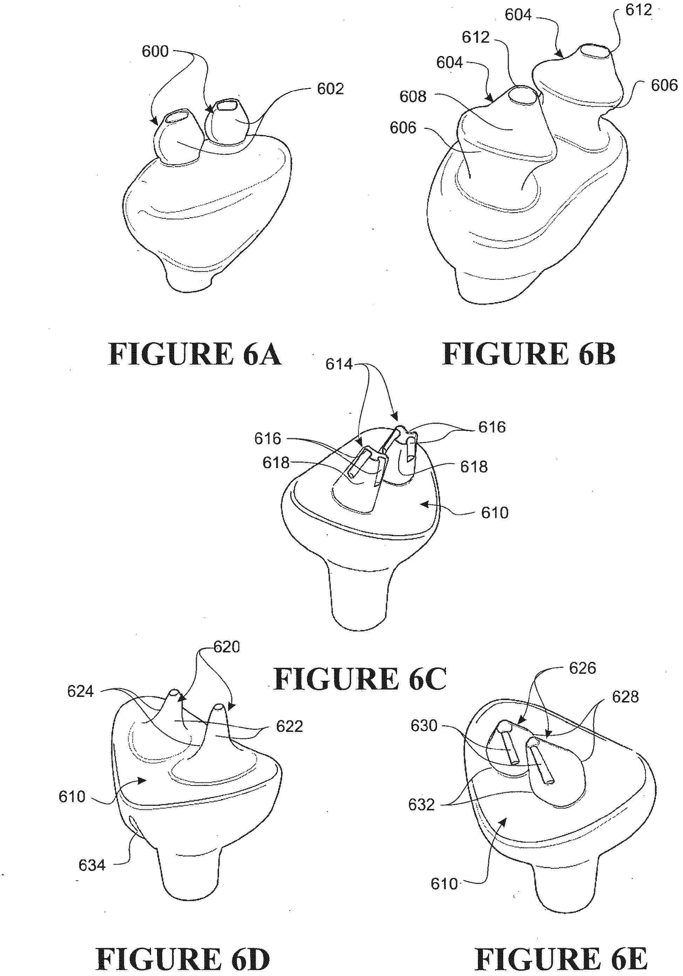

**Figures 6A to 6E** show views of a number of interface variations. The interface variations include variation of the shape of the envelope and the shape of the nasal locators.

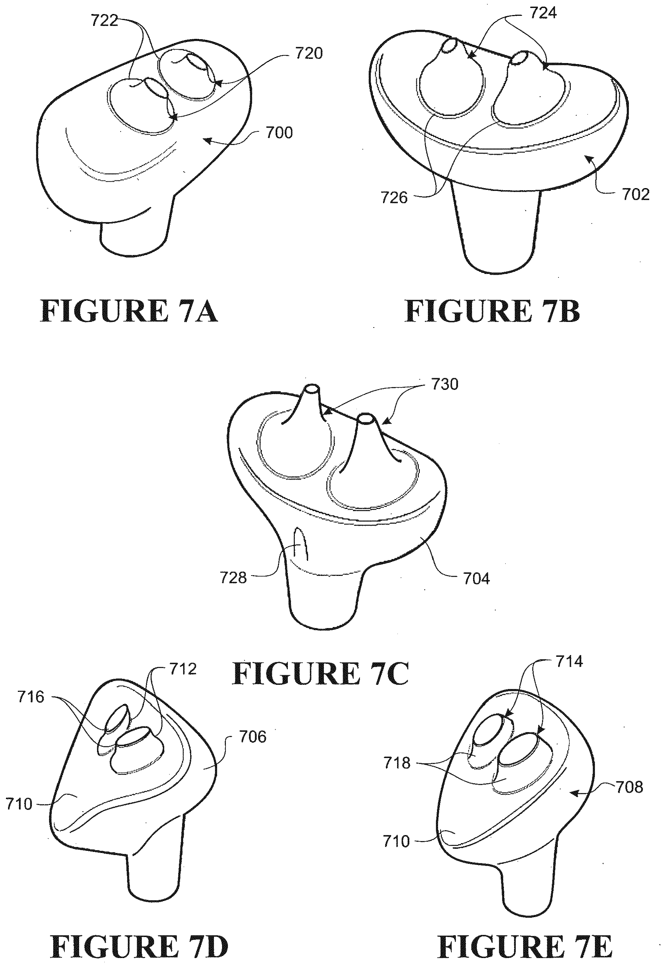

**Figures 7A to 7E** show views of a number of interface variations. The interface variations include variation of the shape of the envelope and the shape of the nasal locators.

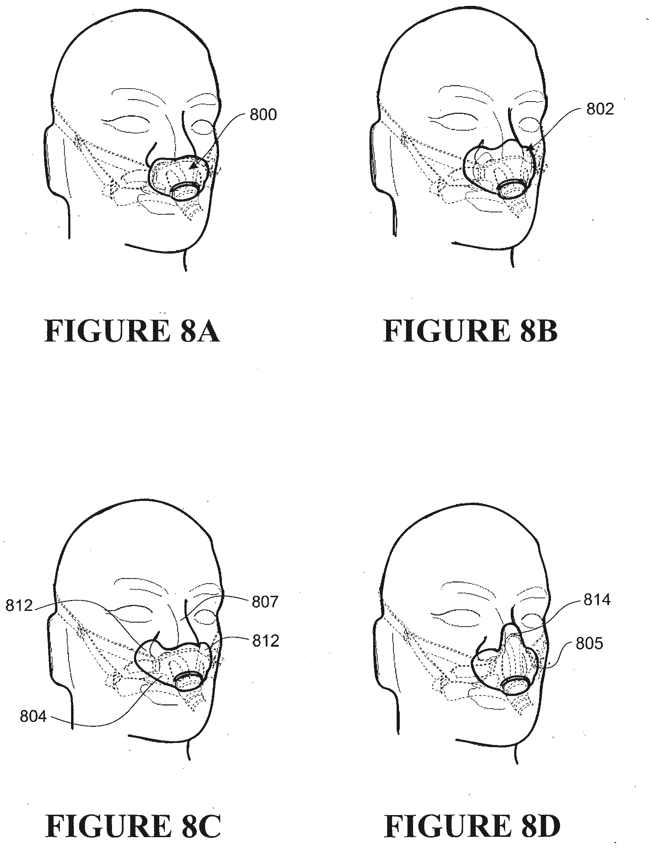

**Figures 8A to 8G** show views of a number of interface variations. The interface variations include variation of the shape of the envelope, which are illustrated relative to features of a user's face.

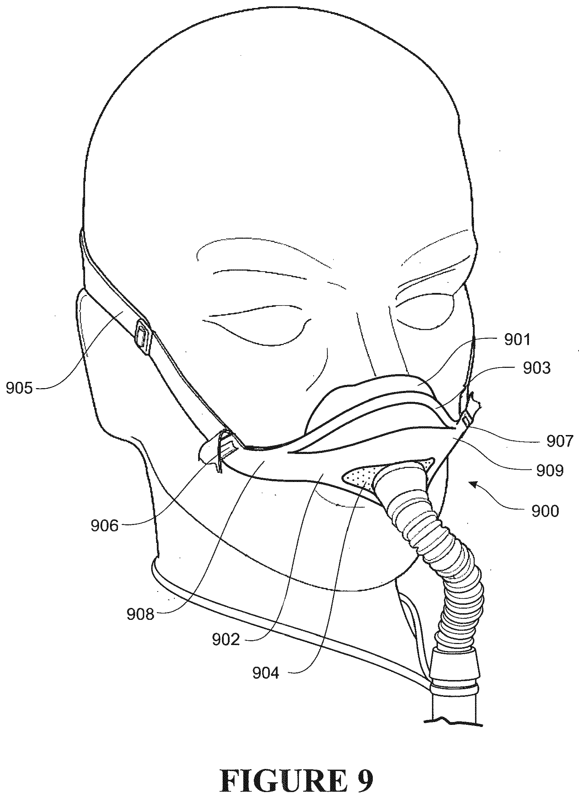

**Figure 9** shows an interface with an alternative frame with a stabilising strap.

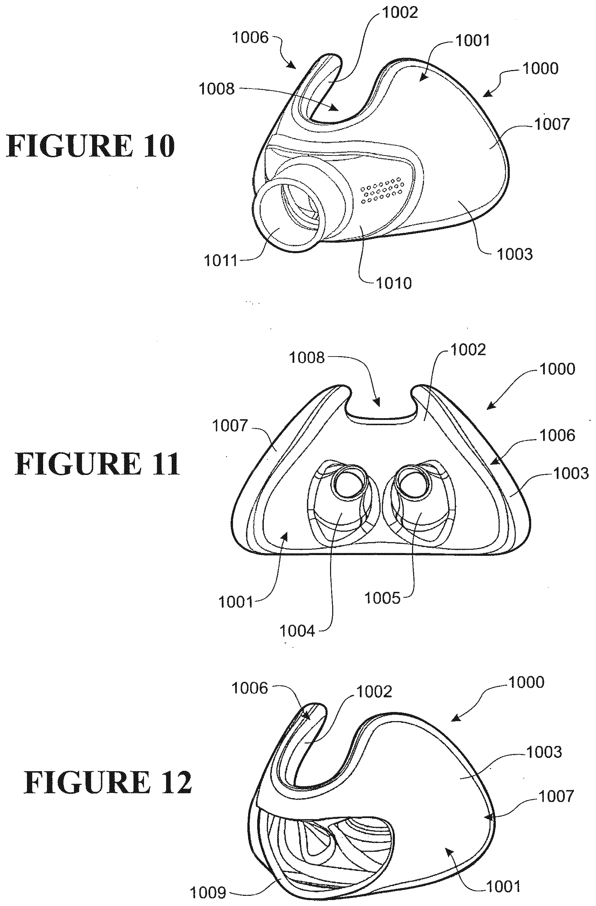

**Figures 10 and 11** show views of a further embodiment of an interface with an inflatable envelope and frame. The envelope is shaped to wrap around the user's nose in use.
**Figure 12** shows the envelope of Figures 10 and 11 with the frame removed.

### DETAILED DESCRIPTION

The present technology provides an interface that includes an inflatable envelope (that could also be referred to as a bag or balloon) having a supple wall structure. The inflatable envelope has a pair of locating protrusions that engage in the nostrils of the user. The locating protrusions supply gases flow to the user from inside the envelope. The envelope is so supple, and of sufficient dimension and shape, that when the inflated envelope is pressed against the face of a user, with the locating protrusions engaged in the nostrils of the user, the envelope contacts the surfaces of the user's face (the nose, the upper lip and the cheeks) and provides a seal.

One example of the interface will be described in detail. Many variations are possible to both the seal part of the interface and to the supporting frame and straps. For example the seal part of the interface could be supported by the type of frames and straps that have been described previously for use with nasal masks, or nasal pillows interfaces. Some variations are illustrated in Figures 6 to 8.

Figure 1 is a view from above of an example interface including inflatable envelope, supporting frame, and straps. Figure 2 is an illustration showing the interface of Figure 1 in position on the head 202 of a user.

The interface includes a seal body 100. The seal body 100 includes a supple envelope 102. When inflated the supple envelope 102 has a general blob shape with a substantially uniform aspect ratio. The envelope has a low wall thickness. The envelope is formed from a material having sufficient elasticity and yield strength that the combination renders the envelope supple. The envelope is capable of repeated drastic deformations without failure. Possible materials include latex, vinyl, silicone and polyurethane. Typically wall thickness would be below 0.5mm and could be lower than 0.2mm.

The seal body includes a pair of nostril locators 104 protruding from the envelope. Preferably the nostril locators are formed integral with the envelope. Each nostril locator includes an outlet aperture for supplying gas from inside the envelope to a user wearing the interface.

The seal body may broadly be shaped to have a sidewall 108 and a seal surface 110. The sidewall 108 extends all around the body. In use either or both of the sidewall 108 and the seal surface 110 may press against surfaces of the user's face to form a seal that conforms around the nose and surrounds the nostril locators. The nostril locators may or may not be formed to also seal with the nostrils of the user.

The seal body 100 includes an inlet opening at the base, approximately opposite the nostril locators 104.

The seal body may be formed, for example, by coating over a male mould. Coating may be performed by spraying the desired material over the male mould in a sequence of coats, or by dipping the male mould into a bath of the desired material one or more times to build up the desired thickness. Apertures in the seal body (for example for the outlet apertures in the nostril locators) may be subsequently cut. The seal body may be pressure tested prior to cutting the apertures.

A substantial extent of the seal body or envelope is supple. This is except for a region immediately adjacent and including the nostril locators and immediately adjacent and including the inlet opening. These areas must have some shape and can be of any suitable stiffness. It is preferred that these areas are stiffer and as such they may be formed of a thicker material, or simply formed thicker in the same material as the rest of the envelope.

The envelope may be so supple that when not pressurised with gases the envelope, bag or balloon hangs limp and does not hold its shape. However, in some embodiments it is required that the envelope retain some shape when not in use (not pressurised). Therefore, the envelope may have some natural shape. For an example, see the embodiment of Figures 10 to 12 .

The seal body 100 is supported by a frame 112. The inlet opening of the seal body is fitted to the frame, or directly to a conduit extending through the frame.

The frame 112 is preferably a minimal design to provide little visual obstructions, allowing a clear field of view and allowing the user to wear glasses while wearing the interface. Preferably the frame is formed from an elastomeric material. This allows the frame to flex to conform slightly to the user's face, while still providing support for the seal body to keep the seal effectively against the face and around the nose of the user.

The frame may be formed by injection moulding, preferably from an elastomeric material such as silicone or polyurethane. However, more rigid materials may be used.

The frame presses the inflated seal body against the face of the user right out to the periphery of the frame. For example a nasal ridge of the frame may press the uppermost periphery 200 of the compressed seal against the ridge 216 of the nose of the user. Side peripheral portions of the frame press side peripheral portions of the seal body against the lower cheeks of the user.

The preferred frame includes connection points for connecting straps 126 to the frame. The strap attachment points provide for anchoring the straps, and preferably for adjusting the strap lengths.

In other forms the envelope may include integral strap attachment points. These attachments may be points on the surface of the envelope. However, they could be integral straps or wings formed in the envelope that extend out either side of the envelope. The strap attachment points are similar to those described above in Figure 1 or 2, but formed on the envelope.

The interface is intended to be supported by a single strap 128 passing around the back of the head. To improve stability of the frame on the face the straps 126 are bifurcated at the forward ends of strap 128. Each end of strap 128 divides at location 134 into an upper strap 130 and a lower strap 132. The straps may be formed from an elastic or elastomeric material. For example suitable strap materials may include a woven elastic strip or a narrow strip of foam and fabric, such as Breathoprene^{™}. The strap extending around the back of the head provides pressure on the mask and helps seal the envelope to the user's face.

The connection points on the frame include lower connection points 122 for receiving lower strap ends 132, and upper connection points 120 for receiving upper strap ends. The connection between frame and strap may be by any suitable buckle, strap bar or clip. In the illustrated embodiment the connection points are provided close to the extreme side limits of the frame.

In the illustrated embodiment of Figures 1 and 2 the frame includes laterally extended supporting arms 124 that can press on the lower cheek portions 214 of the user, possibly outside the periphery of the seal body. The side arms 124 are flexible, but stiffer than the seal body or straps.

The lower strap connection points are preferably provided at the extreme ends of the supporting arms.

The support arms may be provided with some cushioning on their inside surface to contact the user's face. The cushioning could be formed from silicone or from a foam material.

A web 218 of elastic material may span between the flexible support arms 214 and the upper connection points 122. The web provides support to the inflated envelope, pushing it against the face to create a mobile seal in the side peripheral region. The web may be thin relative to the rest of the mask frame.

A flexible tube 114 extends from the frame 112. The flexible tube delivers breathable gas. The distal end of the flexible tube 114 connects to the main CPAP delivery tube 212, for example at connector 210. The flexible tube 114 is preferably made of breathable material such as that tube construction described in US6,769,431 and US7,140,366, except the tube 114 is an inspiratory conduit not expiratory limb.

A lanyard 204 may be connected to the distal end of the flexible tube 114, the connector 210 or the delivery tube 212. The lanyard is to be connected to the body of the user of the interface. For example the lanyard may be worn around the neck 206 of the user. The lanyard carries the weight of the main delivery tube allowing less pressure to hold the interface in place. This may allow a single head strap to hold the interface in place.

The lanyard may connect to the conduit, for example, by a clip 208 that clips into a corrugation of the conduit. Preferably the lanyard or tether is connected to the conduit at a location between 10cm and 50cm from the frame.

A connector 116 may connect the tube and the frame. The connection mechanism may be any suitable connection. This could include a snap fit, hooks in to the silicone, keyhole inserts, over moulding, insert moulding, screw attachments or gluing, or any combination.

The connector 116 may include a limited flow outlet (or bias flow outlet) 220 for providing gas washout from the interface. The outlet 220 may be in the form of a collection of small apertures in the connector. Internally the connector may include a funnel or extension leading from the vent into the mouth of the envelope.

The frame may have any suitable arrangement for securing the envelope. An example is an annular wall extending from the inside of the frame around the perimeter of an opening that extends to the connector 116. The wall includes an outwardly extending lip. The inlet opening of the envelope engages over the outwardly extending lip of the wall. The inlet opening of the envelope is preferably stretched to fit over the lip. The inlet opening of the envelope may be provided with a thickened or reinforced wall section, for example by rolling up an extended section of the envelope.

Alternatively the envelope may be fitted with one part of a connector (for example by adhesive or over moulding), and the frame of the end of the conduit may include a complimentary connector portion.

The frame could be varied substantially and still provide suitable support for the inflatable envelope. The frame could include fewer or additional supporting arms against the face. The frame could incorporate rigid bodies to provide better support. The frame could incorporate a swivel or ball joint for the flexible conduit to allow rotation through different angles and orientations. The frame could include mechanisms to adjust size or shape or both to different users. The frame could support more than one inflatable envelope.

The interface could include more straps to provide support around the user's head. The frame and straps could be moulded from a continuous body of elastomeric material.

Figures 3A to 3D are a sequence of illustrations showing inflation of the inflatable envelope.

Figure 3A shows the seal 300 limp and deflated. The side wall and seal portion of the seal body are collapsed toward the inlet perimeter 308.

Figures 3B shows the envelope 302 slightly inflated.

Figures 3C shows the envelope 304 further inflated.

Figure 3D shows the envelope 306 inflated to substantially the final form in which the internal pressure supports the shape against the effect of gravity. This shows the nostril locators 104 protruding and aligned, ready for positioning in the user's nostrils as the first step in donning the interface.

When inflated without stretch the envelope will not pass through a circular aperture of 40mm diameter without contact.

When the envelope is inflated it holds its shape with an internal pressure equivalent to 3cm H₂O.

The next stage in donning the interface is to press the frame toward the face to compress the seal body around the region of the nose. The user then passes the strap over their head to hold the frame in place, pressing against the seal body.

In use the frame may move relative to the head of the user. This may result from movements of the user during sleep changing the shape of their face or the orientation (and therefore gravity effects) of the interface, forces on the conduit connected to the interface tugging at the frame, or forces directly applied to the frame or straps.

Figures 4A to 4C show how the envelope retains a seal despite substantial vertical movement of the frame relative to the head. The wall thickness of the seal body is exaggerated for clarity. The seal is retained by the seal body conforming to the user's face to retain a sealing area. The seal body conforms to the user's face due to the suppleness and extent of the envelope, together with the treatment pressure inside the seal body pressing the seal body against the facial contours.

Figure 4A shows an intended position 402 of the seal body relative to the user's face. The position and orientation of the frame (which is not shown) is indicated by the inlet 400 of the seal body. The upper seal portion 424 of the seal body is wrapped around the tip of the nose 418. The lower seal portion 422 of the seal body is pressed against the upper lip of the user.

Figure 4B shows a less than ideal position 404 of the seal body relative to the user that might occur if the frame was moved upward as indicated by arrow 408. The more ideal position 402 is shown in ghost for comparison. The upper wall seal and wall portion 428 has rolled upward. The seal now extends to a higher location 416 on end of the nose. The lower wall seal portion 426 has pealed somewhat away from the upper lip of the user, but the abundant supple material still maintains a seal just below the nose at 414.

Figure 4C shows a less than ideal position 406 of the seal body relative to the user that might occur if the frame was moved downward as indicated by arrow 410. The more ideal position 402 is shown in ghost for comparison. The upper seal portion 432 has rolled upward, pealing way from the tip of the nose, but the abundant supple material still maintains a seal at 420. The lower wall seal portion 430 now covers the upper lip, and maintains a seal at 412.

Figures 5A to 5C show how the envelope retains a seal despite substantial lateral movement of the frame relative to the head. The wall thickness of the seal body is exaggerated for clarity. The seal is retained by the seal body conforming to the user's face to retain a sealing area. The seal body conforms to the user's face due to the suppleness and extent of the envelope, together with the treatment pressure inside the seal body pressing the seal body against the facial contours.

Figure 5A shows an intended position 500 of the seal body relative to the user's face. The position and orientation of the frame (which is not shown) is indicated by the inlet 400 of the seal body. The left and right side seal body portions 510 and 520 respectively cover the cheeks to an equal extent on each side of the nose.

Figure 5B shows a less than ideal position 502 that might occur if the frame was moved to the user's right, as indicated by arrow 506. The left side portion 510 of the seal has peeled away from the face at region 526, but still retains a seal in the region of the nares. The right side portion of the seal has rolled out to cover additional area 522 of the right cheek of the user.

Figure 5C shows a less than ideal position 504 that might occur if the frame was moved to the user's right, as indicated by arrow 508. The right side portion of the seal has peeled away from the face at region 524, but still retains a seal in the region of the nares. The left side portion 510 of the seal has rolled out to cover additional area 512 of the left cheek of the user.

One inflating seal according to the present technology has been particularly described above. A range of variations, are illustrated in Figures 5 to 8. These variations display a range of individual features that may be selected and combined in many combinations. The combinations illustrated are selected only for exemplification and other combinations are also possible.

Figures 6A to 6E show views of a number of interface variations. The interface variations include variation of the shape of the envelope and the shape of the nasal locators.

The seal body of Figure 6A includes nasal locators that are shaped to locate in the nares. The locators 600 include a bulge 602 between where they join the envelope and their outlet ends. The bulge helps secure the locators within the nares on inflation with internal air pressure. The narrower base where they join the envelope increases flexibility.

The seal body of Figure 6B includes nasal locators that are shaped to locate against the nares. The locators 604 include a flange between where they join the envelope and their outlet ends. The flange is broader than the nares opening and presses against the lower surfaces of the nose. The flange is pressed against the nares by inflation with internal air pressure. The narrower base where they join the envelope increases flexibility. The narrower tip portion 608 locates inside the nares to locate the seal body.

The seal body of Figure 6C includes nasal locators 614 that are shaped to locate in the nares. The locators 614 include an alternating series of gussets 616 and ridges 618 adjacent their outlet ends. The gussets assist the locators to expand on inflation with internal air pressure.

The seal body of Figure 6D includes nasal locators that are shaped to locate in the nares. The locators 620 have a wide base 624 adjacent the envelope and taper to a narrow tip 622. The narrow tip allows for an easy fit into the nares. The flared base portion 624 provides a comfortable transition to seal in the nares.

The seal body of Figure 6D also illustrates the possibility of providing one or more areas 634 of the envelope shaped to deform in a controlled manner when the seal is compressed against the user's face by the frame. Preferably these areas deform in a predictable way that assists the seal to maintain seal as the frame moves relative to the face.

The seal body of Figure 6E includes nasal locators that are shaped to locate in the nares. The locators 626 include a bulge 628 between where they join the envelope and their outlet ends. The bulge helps secure the locators within the nares on inflation with internal air pressure. The narrower base 632 where they join the envelope increases flexibility. The locators 626 also include one or more gussets 630 to assist the locators to expand when inside the nostrils..

The seal bodies of Figures 6C to 6D have a substantially triangular seal surface 610 to increase the amount of supple seal material available to cover the user's nose. The seal bodies of Figures 6A and 6B have a more oblong or oval shaped seal surface.

Figures 7A to 7E show views of a number of interface variations. The interface variations include variation of the shape of the envelope and the shape of the nasal locators.

The seal body 700 of Figure 7A includes nasal locators that are shaped to locate in and against the nares. The locators 720 have a wide flange 722 adjacent the envelope and taper to a narrower tip. The tip fits into the nares. The flared portion 722 presses against the lower surfaces of the nose. The nasal locator may have a short stalk recessed inside the envelope. The stalk provides flexibility.

The seal body 702 of Figure 7B includes nasal locators that are shaped to locate in and against the nares. The locators 724 have a wide flange 726 adjacent the envelope and taper to a narrower tip. The tip of locator 724 is narrower than the tip of locator 720 of Figure 7A. The narrow tip fits into the nares. The flared portion 722 presses against the lower surfaces of the nose. The nasal locator may have a short stalk recessed inside the envelope. The stalk provides flexibility.

The seal body 704 of Figure 7C is like the seal body of Figure 6D except for an ovoid form rather than a triangular form. The locators 730 have a wide base adjacent the envelope and taper to a narrow tip. The narrow tip allows for an easy fit into the nares. The flared base portion provides a comfortable transition to seal in the nares.

The seal body 706 of Figure 7D includes nasal locators that are shaped to locate in the nares. The locators 712 are gently flared to their base. The locators 712 have oval outlet apertures 716, which match the typical anatomic shape of the nostril entrance. The ovals may be angled relative to each other. The flared base portion may be steeper on the inward facing wall and than the outward wall.

The seal body 708 of Figure 7E is like that of Figure 7D. The nasal locators that are shaped to locate in the nares. The locators 714 are gently flared to their base. The locators 718 have oval outlet apertures 716, which match the typical anatomic shape of the nostril entrance for a larger nose. The ovals may be angled relative to each other. The flared base portion may be steeper on the inward facing wall and than the outward wall.

The seal body of Figure 7C also illustrates the possibility of providing one or more areas 728 of the envelope shaped to deform in a controlled manner when the seal is compressed against the user's face by the frame. Preferably these areas deform in a predictable way that assists the seal to maintain seal as the frame moves relative to the face.

The seal bodies of Figures 7D and 7E have a substantially triangular seal surface 710 to increase the amount of supple seal material available to cover the user's nose. The seal bodies of Figures 7A to 7C have a more oblong or oval shaped seal surface.

Figures 8A to 8G show views of a number of interface variations. The interface variations include variation of the shape of the envelope, which are illustrated relative to features of a user's face, in the form they would take under compression by the interface frame.

The envelope 800 of Figure 8A is shaped to have a smaller overall size which seals generally around the bottom of the nose and the nares. The small minimal form still conforms to the user's face due to the CPAP delivery pressure inside the body. However, the limited lateral and vertical extent of the envelope means it will not retain seal through as large displacement as others illustrated.

The envelope 802 of Figure 8B is shaped to have a larger overall size to encompass more of the area around the nose. The larger extent of the envelope suggests it will retain seal through larger displacements than the seal of Figure 8A.

The envelope 804 of Figure 8C is shaped to have a larger lateral size to accommodate users with wider noses and provide more lateral support. The larger lateral extent 812 of the envelope suggests it will retain the seal through larger lateral displacements than the seal of Figure 8A. Also, the lack of seal over the user's nasal bridge 807 means there is no pressure on the user's bridge, increasing the user's comfort in that area.

The envelope 805 of Figure 8D is shaped to be more upwardly extended, with a definite protruding portion 814. The protruding portion 814 wraps over the nose tip and around the sides of the nose to accommodate longer noses.

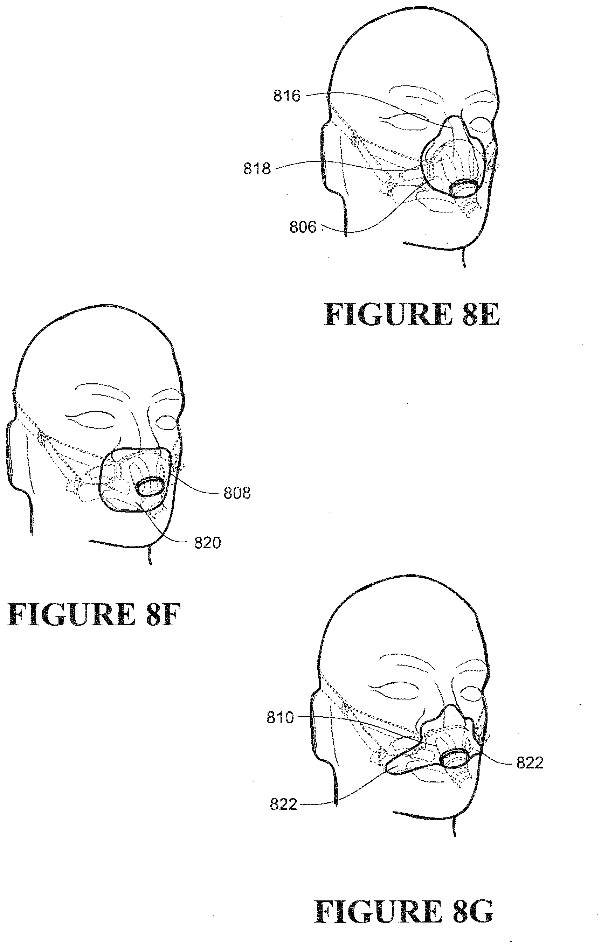

The envelope 806 of Figure 8E is sized to encompass the whole nose having more vertical extent 816 and more lateral extent 818 than the envelope of Figure 8A. This envelope may require a larger and more extensive frame to provide a wide seal.

The envelope 808 of Figure 8F includes a downwardly extending portion 820 that covers the moth of the user. This downward extension may include an oral interface portion for supplying therapy orally as well as, or instead of, nasally.

The envelope 810 of Figure 8G includes wing portions 822 that exemplify the possibility that the seal body could provide cushioning under extended parts of the frame. For example the wing portions could provide cushioning under the support members 124 of the frame of Figure 1.

These variations of envelope shape and variations of nostril locating member are provided for the purpose of illustrating a range of possibilities that could be applied to the inflatable seal.

Figure 9 shows a further alternative embodiment of an interface. The interface 900 has a seal body and supple envelope 901 of any of the embodiments described above.

The envelope 901 has a frame supporting it. The frame 902 cups about the envelope 901. Extending above the frame 902 and envelope 901 is a strap. The strap 903 further stabilises the envelope 901. Particularly, the strap prevents the envelope extending out too far from the user's face and causes the envelope to be pushed against the user's face and more particularly the user's nose. The strap is made from a flexible material similar to that of the frame, but can be made with any stiffness as required. Therefore, a more stiff material could be used, or a substantially more flexible material than the frame.

The frame has bias flow holes 904 formed about a connector for the conduit, providing gases to the interface. The bias flow holes 904 provide gas washout from the interface.

A single head strap 905 extends about the user's head in use. The head strap 905 attaches on either side of the user's head to connectors 906, 907 formed in the frame. The connectors 906, 907 are formed in laterally extending support arms 908, 909 of the frame.

In other forms the strap 903 may be formed in the envelope 901. In this form, the strap is integrally formed in the envelope and provides additional stability to the envelope. Therefore, in effect the integral strap forms an elongate strip in the envelope, the strip extending in an arc above the frame.

Figures 10 to 12 show a further example of an interface with an inflatable envelope, bag or balloon. The interface includes a seal body 1000. The seal body 1000 includes a supple envelope 1001. The seal body 1000 has a curved shape to match the contours of a human face. The seal body 1000 has an inner surface 1002 and an outer surface 1003. The inner surface 1002 includes nostril locators 1004, 1005. The nostril locators 1004, 1005 each have outlets to allow gases to pass to the user. In use, the inner surface 1002 presses against the user's face and the nostril locators 1004, 1005 extend into or sit about the user's nostrils.

The seal body 1000 is shaped to wrap around the user's nose. The seal body 1000 has side portions or wings 1006, 1007 that extend completely over the sides of the user's nose and may also extend at least partially over the user's cheeks. The seal body 1000 is missing in the region of the user's nasal bridge. So effectively, there is a cut out region 1008 in the seal body. The cut out region 1008 means in use the seal body does not put pressure on the user's nasal bridge region, a common area of complaint by users of interfaces that extend over a user's nasal bridge.

The seal body 1000 includes an inlet opening 1009. The inlet opening 1009 is opposite the nostril locators and receives a frame 1010. The frame 1010 is curved to match the seal body and provides support to the seal body. The frame 1010 includes a connector 1011 that attaches in use to the tubing (for example tubing 114 as shown in Figures 1 and 2) that receives pressurised gases for supply the user.

The frame 1010 is made from a plastics material. The plastics material may be a flexible material or maybe a more rigid type material. For example, the frame may be made from a polycarbonate. Or the frame could be made from a more flexible material such as silicone.

In use, the interface of Figures 10 to 12 is supplied via tubing and the connector 1011 on the frame 1010 with pressurised gases. The gases inflate the seal body 1000 and cause it to press against and about the user's nose. The nostril locators 1004, 1005 are caused to seal in or about the user's nostrils and the pressurised gases pass through the locator outlets into the user's nostrils.

The frame or seal body includes at least two strap connectors similar to those described above. The connectors allow a head strap to be attached to the interface 1000. The head strap will extend about the back of the user's head and provide additional tension on the interface to help with the sealing of the interface on the user's face.

The seal body is made from a flexible material. Examples of possible materials include latex, vinyl, silicone and polyurethane. In a preferred form the seal body's outer surface is made from a thicker material than the inner surface. In this way the seal body has a better ability to hold a predetermined shape.

## Claims

1. An interface for providing a supply of pressurised gas to a user, the interface comprising a seal body (100, 700, 702, 704, 706, 708, 1000), a frame (112), and a single strap (128), wherein:
the seal body (100, 700, 702, 704, 706, 708, 1000) comprises a supple envelope (102, 300, 302, 304, 306, 800, 802, 804, 805, 810, 1001) and comprises a sidewall (108) extending all around the seal body (100, 700, 702, 704, 706, 708, 1000), a seal surface (110, 610, 710) and an inlet opening (400, 1009), wherein either or both of the sidewall (108) and the seal surface (110, 610, 710) are configured to, in use, press against surfaces of the user's face to form a seal that conforms around the user's nose,
the seal body (100, 700, 702, 704, 706, 708, 1000) is supported by the frame (112), and the inlet opening (400, 1009) is fitted to the frame (112) or directly to a conduit extending through the frame (112),
the frame (112) comprises laterally extended supporting arms (124) formed in the frame (112) and configured to, in use, press on the lower cheek portions of the user outside the periphery of the seal body, and connection points (120, 122) formed in the laterally extended supporting arms (124) for connecting the strap (128) to the frame (112), wherein, in use, the strap (128) extends around the back of the user's head and provides pressure on the frame (112) and helps seal the seal body (100, 700, 702, 704, 706, 708, 1000) to the user's face,
the laterally extended supporting arms (124) are flexible but stiffer than the seal body (100, 700, 702, 704, 706, 708, 1000) or the strap (128),
the strap (128) is bifurcated at forward ends of the strap (128) to divide into an upper strap (130) and a lower strap (132), and
the connection points (120, 122) comprise a lower connection point (122) for receiving the lower strap (132) and an upper connection point (120) for receiving the upper strap (130), the connection points (120, 122) being provided close to the extreme side limits of the frame (112).

2. The interface of claim 1, wherein the supple envelope (102, 300, 302, 304, 306, 800, 802, 804, 805, 810, 1001) does not seal over the user's nasal bridge (807), thus there is no pressure on the user's nasal bridge (807), increasing the user's comfort in that area.

3. The interface of either of the preceding claims, wherein a substantial extent of the seal body (100, 700, 702, 704, 706, 708, 1000) is supple.

4. The interface of any one of the preceding claims, wherein, in use, the frame (112) provides little visual obstruction, allowing a clear field of view and allowing the user to wear glasses while wearing the interface.

5. The interface of any one of the preceding claims, wherein the frame (112) is configured to flex to conform slightly to the user's face in use.

6. The interface of any one of the preceding claims, wherein the connection points (120, 122) provide for adjusting a length of the strap (128).

7. The interface of any one of the preceding claims, wherein the strap (128) is formed of an elastic material.

8. The interface of any one of the preceding claims, wherein a flexible tube (114) extends from the frame (112) to deliver breathable gas.

9. The interface of claim 8, wherein a connector (116) connects the flexible tube (114) and the frame (112) with a snap fit, and the connector (116) comprises a limited flow outlet (220) in the form of a collection of small apertures in the connector (116) for providing gas washout from the interface.

10. The interface of claim 8 or claim 9, wherein the frame (112) comprises a swivel or ball joint for the flexible tube (114).

11. The interface of any one of the preceding claims, wherein the seal body (100, 700, 702, 704, 706, 708, 1000) further comprises one or more areas (634) configured to deform in a controlled manner when the seal is compressed against the user's face by the frame such that the seal body (100, 700, 702, 704, 706, 708, 1000) maintains a seal with the user's face when the frame (112) moves relative to the user's face.

12. The interface of any one of the preceding claims, wherein the supple envelope (102, 300, 302, 304, 306, 800, 802, 804, 805, 810, 1001) includes a downwardly extending portion (820) that covers the user's mouth, preferably wherein the downwardly extending portion (820) includes an oral interface portion.

13. The interface of any one of the preceding claims, wherein the seal body (100, 700, 702, 704, 706, 708, 1000) is made from a silicone material.

14. The interface of any one of the preceding claims, wherein the supporting arms (124) are provided with some cushioning on their inside surface to contact the user's face.

15. The interface of any one of the preceding claims, wherein the supple envelope (102, 300, 302, 304, 306, 800, 802, 804, 805, 810, 1001) is fitted with a connector portion and a frame of an end of a conduit includes a complementary connector portion for securing the supple envelope to the conduit.

16. The interface of any one of the preceding claims, wherein the frame can support more than one supple envelope (102, 300, 302, 304, 306, 800, 802, 804, 805, 810, 1001).

17. The interface of any one of the preceding claims, wherein the seal body (100, 700, 702, 704, 706, 708, 1000) has a curved shape to match the contours of a human face, and is shaped to wrap around the user's nose.

18. The interface of any one of the preceding claims, wherein the supple envelope (102, 300, 302, 304, 306, 800, 802, 804, 805, 810, 1001) has a pair of nostril locators (104, 600, 604, 614, 620, 626, 712, 714, 718, 720, 724, 726, 730, 1004, 1005) that engage in respective nostrils of the user, each nostril locator (104, 600, 604, 614, 620, 626, 712, 714, 718, 720, 724, 726, 730, 1004, 1005) including an outlet aperture (716) for supplying gas from inside the supple envelope (102, 300, 302, 304, 306, 800, 802, 804, 805, 810, 1001) to the user wearing the interface.

## Patentansprüche

1. Schnittstelle zur Bereitstellung einer Versorgung mit druckbeaufschlagtem Gas an einen Benutzer, wobei die Schnittstelle einen Dichtungskörper (100, 700, 702, 704, 706, 708, 1000), einen Rahmen (112) und einen einzelnen Riemen (128) umfasst, wobei:
der Dichtungskörper (100, 700, 702, 704, 706, 708, 1000) eine biegsame Hülle (102, 300, 302, 304, 306, 800, 802, 804, 805, 810, 1001) umfasst und eine sich um den gesamten Dichtungskörper (100, 700, 702, 704, 706, 708, 1000) erstreckende Seitenwand (108), eine Dichtungsfläche (110, 610, 710) und eine Einlassöffnung (400, 1009) umfasst, wobei entweder die Seitenwand (108), die Dichtungsfläche (110, 610, 710) oder beide dazu ausgebildet sind, im Gebrauch gegen Oberflächen des Gesichts des Benutzers zu drücken, um eine Dichtung zu bilden, die sich um die Nase des Benutzers legt,
der Dichtungskörper (100, 700, 702, 704, 706, 708, 1000) durch den Rahmen (112) getragen wird und die Einlassöffnung (400, 1009) am Rahmen (112) oder direkt an einer sich durch den Rahmen (112) erstreckenden Leitung angebracht ist,
der Rahmen (112) seitlich ausgedehnte Stützarme (124), die im Rahmen (112) gebildet sind und dazu ausgebildet sind, im Gebrauch auf die unteren Wangenabschnitte des Benutzers außerhalb des Umfangs des Dichtungskörpers zu drücken, und Verbindungspunkte (120, 122) umfasst, die in den seitlich ausgedehnten Stützarmen (124) gebildet sind, zum Verbinden des Riemens (128) mit dem Rahmen (112), wobei sich im Gebrauch der Riemen (128) um die Rückseite des Kopfes des Benutzers erstreckt und Druck auf den Rahmen (112) ausübt und dazu beiträgt, den Dichtungskörper (100, 700, 702, 704, 706, 708, 1000) gegenüber dem Gesicht des Benutzers abzudichten,
die seitlich ausgedehnten Stützarme (124) flexibel, aber steifer als der Dichtungskörper (100, 700, 702, 704, 706, 708, 1000) oder der Riemen (128) sind,
der Riemen (128) an vorderen Enden des Riemens (128) gegabelt ist, um sich in einen oberen Riemen (130) und einen unteren Riemen (132) zu teilen, und
die Verbindungspunkte (120, 122) einen unteren Verbindungspunkt (122) zum Aufnehmen des unteren Riemens (132) und einen oberen Verbindungspunkt (120) zum Aufnehmen des oberen Riemens (130) umfassen, wobei die Verbindungspunkte (120, 122) nahe an den äußersten seitlichen Begrenzungen des Rahmens (112) bereitgestellt sind.

2. Schnittstelle nach Anspruch 1, wobei die biegsame Hülle (102, 300, 302, 304, 306, 800, 802, 804, 805, 810, 1001) nicht über dem Nasenrücken (807) des Benutzers abdichtet, so dass kein Druck auf den Nasenrücken (807) des Benutzers ausgeübt wird, wodurch der Komfort des Benutzers in diesem Bereich erhöht wird.

3. Schnittstelle nach einem der beiden vorstehenden Ansprüche, wobei ein erheblicher Umfang des Dichtungskörpers (100, 700, 702, 704, 706, 708, 1000) biegsam ist.

4. Schnittstelle nach einem der vorstehenden Ansprüche, wobei der Rahmen (112) im Gebrauch nur eine geringe Sichtbehinderung verursacht, wodurch ein freies Sichtfeld ermöglicht wird und wodurch es dem Benutzer ermöglicht wird, eine Brille zu tragen, während er die Schnittstelle trägt.

5. Schnittstelle nach einem der vorstehenden Ansprüche, wobei der Rahmen (112) im Gebrauch dazu ausgebildet ist, sich zu biegen, um sich geringfügig an das Gesicht des Benutzers anzupassen.

6. Schnittstelle nach einem der vorstehenden Ansprüche, wobei die Verbindungspunkte (120, 122) ein Einstellen einer Länge des Riemens (128) ermöglichen.

7. Schnittstelle nach einem der vorstehenden Ansprüche, wobei der Riemen (128) aus einem elastischen Material gebildet ist.

8. Schnittstelle nach einem der vorstehenden Ansprüche, wobei sich ein flexibler Schlauch (114) vom Rahmen (112) erstreckt, um Atemgas zuzuführen.

9. Schnittstelle nach Anspruch 8, wobei ein Verbinder (116) den flexiblen Schlauch (114) und den Rahmen (112) mittels Schnappverbindung verbindet und der Verbinder (116) eine begrenzte Durchflussauslassöffnung (220) in Form einer Ansammlung kleiner Öffnungen im Verbinder (116) zum Bereitstellen einer Gasabführung aus der Schnittstelle umfasst.

10. Schnittstelle nach Anspruch 8 oder Anspruch 9, wobei der Rahmen (112) ein Dreh- oder Kugelgelenk für den flexiblen Schlauch (114) umfasst.

11. Schnittstelle nach einem der vorstehenden Ansprüche, wobei der Dichtungskörper (100, 700, 702, 704, 706, 708, 1000) weiter einen oder mehrere Bereiche (634) umfasst, die dazu ausgebildet sind, sich in kontrollierter Weise zu verformen, wenn die Dichtung durch den Rahmen gegen das Gesicht des Benutzers zusammengedrückt wird, so dass der Dichtungskörper (100, 700, 702, 704, 706, 708, 1000) eine Dichtung mit dem Gesicht des Benutzers aufrechterhält, wenn sich der Rahmen (112) relativ zum Gesicht des Benutzers bewegt.

12. Schnittstelle nach einem der vorstehenden Ansprüche, wobei die biegsame Hülle (102, 300, 302, 304, 306, 800, 802, 804, 805, 810, 1001) einen nach unten erstreckenden Abschnitt (820) einschließt, der den Mund des Benutzers abdeckt, bevorzugt, wobei der nach unten erstreckende Abschnitt (820) einen oralen Schnittstellenabschnitt einschließt.

13. Schnittstelle nach einem der vorstehenden Ansprüche, wobei der Dichtungskörper (100, 700, 702, 704, 706, 708, 1000) aus einem Silikonmaterial hergestellt ist.

14. Schnittstelle nach einem der vorstehenden Ansprüche, wobei die Stützarme (124) an ihrer Innenseite mit einer Polsterung versehen sind, um das Gesicht des Benutzers zu kontaktieren.

15. Schnittstelle nach einem der vorstehenden Ansprüche, wobei die biegsame Hülle (102, 300, 302, 304, 306, 800, 802, 804, 805, 810, 1001) mit einem Verbinderabschnitt versehen ist und ein Rahmen eines Endes einer Leitung einen komplementären Verbinderabschnitt einschließt, um die biegsame Hülle an der Leitung zu befestigen.

16. Schnittstelle nach einem der vorstehenden Ansprüche, wobei der Rahmen mehr als eine biegsame Hülle (102, 300, 302, 304, 306, 800, 802, 804, 805, 810, 1001) stützen kann.

17. Schnittstelle nach einem der vorstehenden Ansprüche, wobei der Dichtungskörper (100, 700, 702, 704, 706, 708, 1000) eine gekrümmte Form aufweist, um den Konturen eines menschlichen Gesichts zu entsprechen, und so geformt ist, dass er sich um die Nase des Benutzers legt.

18. Schnittstelle nach einem der vorstehenden Ansprüche, wobei die biegsame Hülle (102, 300, 302, 304, 306, 800, 802, 804, 805, 810, 1001) ein Paar Nasenlochlokalisatoren (104, 600, 604, 614, 620, 626, 712, 714, 718, 720, 724, 726, 730, 1004, 1005) aufweist, die in entsprechende Nasenlöcher des Benutzers eingreifen, wobei jeder Nasenlochlokalisator (104, 600, 604, 614, 620, 626, 712, 714, 718, 720, 724, 726, 730, 1004, 1005) eine Austrittsöffnung (716) einschließt, um den die Schnittstelle tragenden Benutzer mit Gas aus dem Inneren der biegsamen Hülle (102, 300, 302, 304, 306, 800, 802, 804, 805, 810, 1001) zu versorgen.

## Revendications

1. Interface pour fournir une alimentation en gaz sous pression à un utilisateur, l'interface comprenant un corps d'étanchéité (100, 700, 702, 704, 706, 708, 1000), un cadre (112) et une sangle (128) unique, dans laquelle :
le corps d'étanchéité (100, 700, 702, 704, 706, 708, 1000) comprend une enveloppe souple (102, 300, 302, 304, 306, 800, 802, 804, 805, 810, 1001) et comprend une paroi latérale (108) s'étendant tout autour du corps d'étanchéité (100, 700, 702, 704, 706, 708, 1000), une surface d'étanchéité (110, 610, 710) et une ouverture d'admission (400, 1009), l'une ou l'autre, ou les deux, de la paroi latérale (108) et de la surface d'étanchéité (110, 610, 710) étant configurées pour, en utilisation, appuyer contre des surfaces du visage de l'utilisateur pour former un joint qui se conforme autour du nez de l'utilisateur,
le corps d'étanchéité (100, 700, 702, 704, 706, 708, 1000) est supporté par le cadre (112), et l'ouverture d'admission (400, 1009) est montée sur le cadre (112) ou directement sur un conduit s'étendant à travers le cadre (112),
le cadre (112) comprend des bras de support (124) s'étendant latéralement formés dans le cadre (112) et configurés pour, en utilisation, appuyer sur les parties inférieures des joues de l'utilisateur à l'extérieur de la périphérie du corps d'étanchéité, et des points de fixation (120, 122) formés dans les bras de support (124) s'étendant latéralement pour relier la sangle (128) au cadre (112), dans laquelle, en utilisation, la sangle (128) s'étend autour de l'arrière de la tête de l'utilisateur et exerce une pression sur le cadre (112) et aide à étanchéifier le corps d'étanchéité (100, 700, 702, 704, 706, 708, 1000) sur le visage de l'utilisateur,
les bras de support (124) s'étendant latéralement sont flexibles mais plus rigides que le corps d'étanchéité (100, 700, 702, 704, 706, 708, 1000) ou la sangle (128),
la sangle (128) est bifurquée au niveau de ses extrémités avant de manière à se diviser en une sangle supérieure (130) et une sangle inférieure (132), et
les points de fixation (120, 122) comprennent un point de fixation inférieur (122) pour recevoir la sangle inférieure (132) et un point de fixation supérieur (120) pour recevoir la sangle supérieure (130), les points de fixation (120, 122) étant prévus à proximité des limites latérales extrêmes du cadre (112).

2. Interface selon la revendication 1, dans laquelle l'enveloppe souple (102, 300, 302, 304, 306, 800, 802, 804, 805, 810, 1001) ne réalise pas d'étanchéité au-dessus du pont nasal (807) de l'utilisateur, de telle sorte qu'il n'y a pas de pression sur le pont nasal (807) de l'utilisateur, ce qui augmente le confort de l'utilisateur dans cette zone.

3. Interface selon l'une quelconque des revendications précédentes, dans laquelle une partie substantielle du corps d'étanchéité (100, 700, 702, 704, 706, 708, 1000) est souple.

4. Interface selon l'une quelconque des revendications précédentes, dans laquelle, en utilisation, le cadre (112) présente peu d'obstruction visuelle, permettant un champ de vision dégagé et permettant à l'utilisateur de porter des lunettes tout en portant l'interface.

5. Interface selon l'une quelconque des revendications précédentes, dans laquelle le cadre (112) est configuré pour fléchir de manière à se conformer légèrement au visage de l'utilisateur en utilisation.

6. Interface selon l'une quelconque des revendications précédentes, dans laquelle les points de fixation (120, 122) permettent de régler une longueur de la sangle (128).

7. Interface selon l'une quelconque des revendications précédentes, dans laquelle la sangle (128) est formée d'un matériau élastique.

8. Interface selon l'une quelconque des revendications précédentes, dans laquelle un tube flexible (114) s'étend depuis le cadre (112) pour fournir un gaz respirable.

9. Interface selon la revendication 8, dans laquelle un raccord (116) relie le tube flexible (114) et le cadre (112) avec un ajustement par encliquetage, et le raccord (116) comprend un orifice de débit limité (220) sous la forme d'un ensemble de petites ouvertures dans le raccord (116) pour assurer une évacuation de gaz depuis l'interface.

10. Interface selon la revendication 8 ou la revendication 9, dans laquelle le cadre (112) comprend un raccord orientable ou un joint à rotule pour le tube flexible (114).

11. Interface selon l'une quelconque des revendications précédentes, dans laquelle le corps d'étanchéité (100, 700, 702, 704, 706, 708, 1000) comprend en outre une ou plusieurs zones (634) configurées pour se déformer de manière contrôlée lorsque le joint est comprimé contre le visage de l'utilisateur par le châssis, de telle sorte que le corps d'étanchéité (100, 700, 702, 704, 706, 708, 1000) maintient un joint avec le visage de l'utilisateur lorsque le cadre (112) se déplace par rapport au visage de l'utilisateur.

12. Interface selon l'une quelconque des revendications précédentes, dans laquelle l'enveloppe souple (102, 300, 302, 304, 306, 800, 802, 804, 805, 810, 1001) comporte une portion s'étendant vers le bas (820) qui recouvre la bouche de l'utilisateur, de préférence, dans laquelle la portion s'étendant vers le bas (820) comporte une portion d'interface orale.

13. Interface selon l'une quelconque des revendications précédentes, dans laquelle le corps d'étanchéité (100, 700, 702, 704, 706, 708, 1000) est réalisé en un matériau en silicone.

14. Interface selon l'une quelconque des revendications précédentes, dans laquelle les bras de support (124) sont pourvus de coussinets sur leur face interne pour venir en contact avec le visage de l'utilisateur.

15. Interface selon l'une quelconque des revendications précédentes, dans laquelle l'enveloppe souple (102, 300, 302, 304, 306, 800, 802, 804, 805, 810, 1001) est pourvue d'une portion de raccord et un châssis d'une extrémité d'un conduit comporte une portion de raccord complémentaire pour assurer la fixation de l'enveloppe souple au conduit.

16. Interface selon l'une quelconque des revendications précédentes, dans laquelle le châssis peut supporter plus d'une enveloppe souple (102, 300, 302, 304, 306, 800, 802, 804, 805, 810, 1001).

17. Interface selon l'une quelconque des revendications précédentes, dans laquelle le corps d'étanchéité (100, 700, 702, 704, 706, 708, 1000) présente une forme courbe pour correspondre aux contours d'un visage humain et est façonné pour entourer le nez de l'utilisateur.

18. Interface selon l'une quelconque des revendications précédentes, dans laquelle l'enveloppe souple (102, 300, 302, 304, 306, 800, 802, 804, 805, 810, 1001) présente une paire de localisateurs de narines (104, 600, 604, 614, 620, 626, 712, 714, 718, 720, 724, 726, 730, 1004, 1005) qui viennent s'engager dans des narines respectives de l'utilisateur, chaque localisateur de narine (104, 600, 604, 614, 620, 626, 712, 714, 718, 720, 724, 726, 730, 1004, 1005) comportant un orifice de sortie (716) pour fournir un gaz depuis l'intérieur de l'enveloppe souple (102, 300, 302, 304, 306, 800, 802, 804, 805, 810, 1001) à l'utilisateur portant l'interface.
